# EUROPÄISCHE PATENTSCHRIFT

(11) **EP 0 828 527 B1**
(45) Veröffentlichungstag und Bekanntmachung des Hinweises auf die Patenterteilung: **31.07.2002**
(21) Anmeldenummer: 96906692.7
(22) Anmeldetag: 02.04.1996
(51) Int. Cl.: A61M 5/315

(54) **INJEKTIONSGERÄT**
INJECTION DEVICE
DISPOSITIF D'INJECTION

(43) Veröffentlichungstag der Anmeldung: 18.03.1998
(73) Patentinhaber: Disetronic Licensing AG, 3401 Burgdorf (CH)
(72) Erfinder: KIRCHHOFER, Fritz, CH-3454 Sumiswald (CH); MICHEL, Peter, CH-3401 Burgdorf (CH)
(74) Vertreter: Schwabe - Sandmair - Marx
(86) Internationale Anmeldenummer: CH9600116
(87) Internationale Veröffentlichungsnummer: WO9736626

(56) Entgegenhaltungen:
- EP-A- 0 373 321
- WO-A-94/15120
- WO-A-96/07443
- FR-A- 2 701 211

## Beschreibung

Die Erfindung betrifft ein Injektionsgerät zum Injizieren einer vorwählbaren Dosis einer flüssigen Substanz aus einer in einem Ampullenhalter befindlichen Ampulle mit einem mit diesem mechanisch verbundenen hülsenförmigen Mechanikhalter, in dessen Innern eine längsverschiebare Schubstange mit einem daran angeordneten Stopfen vorgesehen ist, der auf die Ampulle einwirkt, wobei die Schubstange von einer mit dieser mechanisch gekoppelten, ebenfalls längsverschiebbaren Vorschubhülse umgeben ist, die in ihrem oberen Endbereich von einem Deckel abgeschlossen ist.

FR-A-2 707 211 offenbart ein gattungsgemäßes Injektionsgerät. Zum Laden des Injektionsgerätes wird eine mit einem Markierungspfeil versehene Vorschubhülse herausgezogen, so dass am unteren Ende der Vorschubhülse vorgesehene Sperrklinken über die Verzahnung der Vorschubstange gleiten, bei gleichzeitig festgehaltener Vorschubstange. Ein Mechanismus, der Sperrklinken an dem Mechanikhalter und einen verbreiterten Rand am oberen Ende der Vorschubstange umfasst, verhindert das Laden des Injektionsgeräts durch Herausziehen der Schubstange dann, wenn der Vorrat der Ampulle vollständig aufgebraucht ist.

Dieses Injektionsgerät weist eine Anzeigeeinrichtung für die zu wählende Dosis auf. Ob die tatsächlich noch in der Ampulle vorhandene Restmenge jedoch der voreingestellten Dosis entspricht, kann nicht zuverlässig festgestellt werden, sondern allenfalls an Hand der Anzahl von Klickgeräuschen, wenn die Sperrklinken der Vorschubhülse über die Verzahnung der Vorschubstange zurückgleiten, oder an Hand des Abstands zwischen dem oberen Rand der Vorschubhülse und dem oberen Rand des Mechanikhalters beim Laden des Injektionsgeräts.

WO-A-96/07443 offenbart ein Injektionsgerät mit einer Anzeige zum Einstellen einer zu injizierenden Dosis und mit einem Mechanismus, der ein erneutes Laden des Injektionsgeräts dann verhindert, wenn der Vorrat der Ampulle des Injektionsgeräts vollständig aufgebraucht ist. Eine Anzeige für den tatsächlich noch vorhandenen Restinhalt der Ampulle ist nicht vorgesehen.

Diesen und anderen Injektionsgeraten gattungsmäßiger Art liegt außerdem das Problem zugrunde, dass je nach Dosiswahl, wenn sozusagen der letzte Tropfen, der keiner vollen Dosis entspricht, noch in der Ampulle vorhanden ist, dass dieser Vorrat nicht mehr ausreicht die eingestellte Dosis voll zu verabreichen. Der Bediener hat bislang dafür kein Instrumentarium, zu erkennen, dass diese von ihm voreingestellte Dosis gar nicht mehr vollständig verabreicht werden kann und injiziert sich möglicherweise eine Unterdosis mit gravierenden gesundheitlichen Folgen.

Aufgabe der vorliegenden Erfindung ist es, das gattungsgemäße Injektionsgerät dahingehend weiterzubilden, dass eine höhere Bediensicherheit hinsichtlich der in der Ampulle tatsächlich noch vorhandenen Restmenge gegeben ist. Diese Aufgabe wird durch ein Injektionsgerät mit den Merkmalen gemäß Patentanspruch 1 gelöst. Vorteilhafte Weiterbildungen sind Gegenstand der rückbezogenen Unteransprüche.

Erfindungsgemäß ist zum Vorwählen einer zu injizierenden Dosis eine Dosierhülse mit einem Treppenglied vorgesehen, die am Mechanikhalter in dessen oberen Endbereich angeordnet ist, wobei durch eine Drehbewegung der Dosierhülse mit dem Treppenglied die jeweils zu verabreichende Dosis einstellbar ist, wobei über einen Dosisaufdruck an einem zylindrischen Teil der Dosierhülse im Zusammenwirken mit einem Markierpfeil am Mechanikhalter die eingestellte Dosis ablesbar ist. Gemäß der Erfindung ist die tatsächlich mögliche verabreichbare Dosis an einer im Bereich des oberen Endes der Vorschubhülse angebrachten Dosisskala ablesbar. Durch das Zusammenwirken von einem im unteren Endbereich der Vorschubhülse angeordneten Anschlag mit einem an der Schubstange angeordneten Steg wird ein Mechanismus ausgebildet, der gewährleistet, dass die Vorschubhülse dann nicht mehr beweglich ist, wenn der Vorrat der Ampulle vollständig aufgebraucht ist.

Die Dosierhülse ist dabei zusammengesetzt aus einem Bedienelement mit einer Profilierung, einem daran anschliessenden zylindrischen Teil mit dem Dosisaufdruck, einem Einrastanschlag und dem daran sich anschliessenden Treppenglied. Die konstruktive Anbindung der Dosierhülse an das Injektionsgerät ist dadurch gekennzeichnet, dass die Dosierhülse mit dem Treppenglied die Vorschubhülse umgibt.

Das erfindungsgemässe Injektionsgerät hat den wesentlichen Vorteil, dass eine einmal eingestellte Dosis immer wieder appliziert werden kann; der Benutzer muss vor jeder Injektion das Injektionsgerät nur noch laden. Die Vorwahl der zu applizierenden Dosis ist denkbar einfach durch eine einfache Drehbewegung der Dosierhülse. Ausserdem garantiert dieses Injektionsgerät eine sichere Benutzung bis zum Ampullenende.

Weitere vorteilhafte Ausgestaltungen der Erfindung ergeben sich aus den abhängigen Ansprüchen.

Ein Ausführungsbeispiel der Erfindung ist in den Figuren dargestellt.

Es zeigen:
Fig. 1: das erfindungsgemässe Injektionsgerät im Schnitt;
Fig. 2: das Injektionsgerät in einer Schnittdarstellung, die gegenüber der in Fig. 1 gezeigten Darstellung um 90 Grad gedreht ist;
Fig. 3: das Injektionsgerät in Vorderansicht;
Fig. 4: das Injektionsgerät im Schnitt gemäss Fig. 2 mit Schubstange im Endanschlag;
Fig. 5; eine Schnittdarstellung längs der Linie A - A in Figur 1; und
Fig. 6: eine Schnittdarstellung längs der Linie B - B in Figur 1.

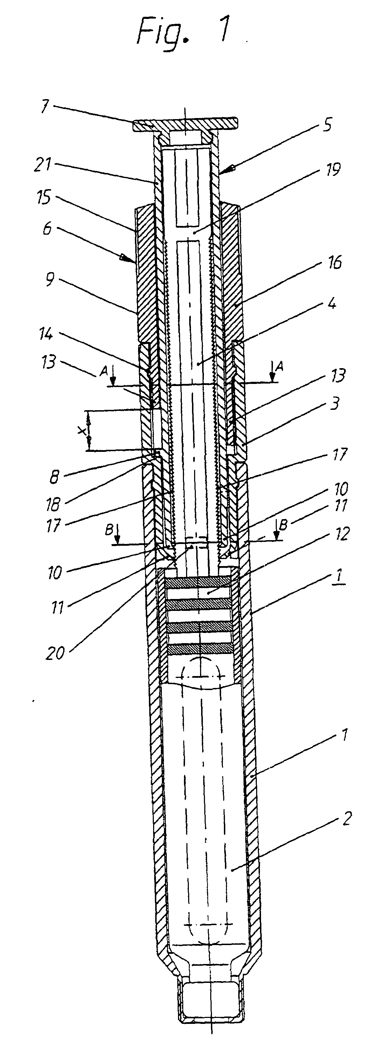

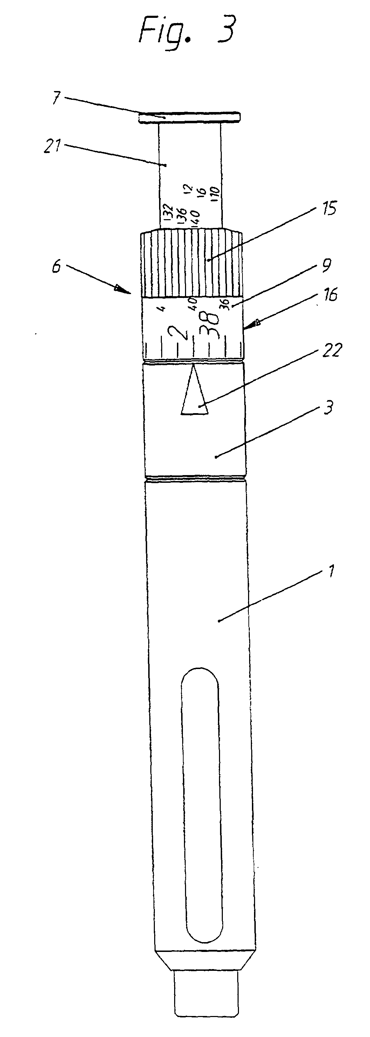

Das in Fig. 1 gezeigte Injektionsgerät besteht aus dem Ampullenhalter 1, in dem sich die Ampulle 2 mit dem Stopfen 12 und mit der zu verabreichenden flüssigen Substanz befindet. Mit dem Ampullenhalter 1 ist der hülsenförmige Mechanikhalter 3 mechanisch verbunden durch ein Gewinde oder durch einen geeigneten anderen Verschluss. Im Innern des Mechanikhalters 3 ist die längsverschiebbare Schubstange 4 vorgesehen, die auf die Ampulle 2 einwirkt. Die Schubstange 4 ist von der mit dieser mechanisch gekoppelten, ebenfalls längsverschiebbaren Vorschubhülse 5 umgeben, die in ihrem oberen Endbereich von dem Deckel 7 abgeschlossen ist. Zum Vorwählen einer zu injizierenden Dosis der flüssigen Substanz ist die Dosierhülse 6 mit dem Treppenglied 13 vorgesehen, die am Mechanikhalter 3 in dessen oberen Endbereich angeordnet ist. Dabei umgibt die Dosierhülse 6 mit dem Treppenglied 13 die Vorschubhülse 5. Durch eine einfache Drehbewegung der Dosierhülse mit dem Treppenglied 13 ist die jeweils einzustellende und zu verabreichende Dosis vorwählbar. Die Dosierhülse 6 ist über den Einrastanschlag 14 mit dem Mechanikhalter 3 mechanisch gekoppelt. Die Dosierhülse 6 besteht aus dem Bedienelement 15 mit Profilierung, dem sich daran anschliessenden zylindrischen Teil 16 mit dem Dosisaufdruck 9, dem Einrastanschlag 14 und dem sich daran anschliessenden Treppenglied 13. Im Zusammenwirken mit dem am Mechanikhalter 3 angebrachten Markierpfeil 22 ist die eingestellte Dosis ablesbar. An der Vorschubhülse 5 ist der Nocken 8 angeordnet, der beim Herausziehen der Vorschubhülse 5 in Richtung des Deckels 7 gegen die jeweils eingestellte Treppenstufe des Treppenglieds 13 anschlägt. Beim Niederdrücken der Vorschubhülse 5 schlägt der Nocken 8 gegen die Schulter 18 des Mechanikhalters 3 an, wodurch sich der Injektionshub X ergibt. Zur mechanischen Kopplung von Schubstange 4 mit der Vorschubhülse 5 ist die Schubstange 4 mit den zwei gegenüberliegenden Verzahnungen 17 versehen, die mit den Rastnocken 10 der Vorschubhülse 5 zusammenwirken. Dabei entspricht die Zahnhöhe der Zähne der Verzahnungen 17 an der Schubstange 4 einer Stufenhöhe des Treppenglieds 13. Der Mechanikhalter 3 weist im Bereich seines unteren Endes die Rasten 11 auf, die ebenfalls mit den Verzahnungen 17 der Schubstange 4 zusammenwirken. Die mittels Dosierhülse 6 mit Treppenglied 13 eingestellte vorwählbare Dosis kann möglicherweise bei einem dafür nicht ausreichenden Restvorrat in der Ampulle 2 nicht vollständig verabreicht werden. Um sich darüber Klarheit zu verschaffen, welche quantitative Menge der flüssigen Substanz tatsächlich noch verabreicht werden kann, ist an der im Bereich des oberen Endes der Vorschubhülse 5 angebrachten Dosisskala 21 die tatsächlich mögliche verabreichbare Dosis ablesbar (Fig. 3). Durch das Zusammenwirken von dem im unteren Endbereich der Vorschubhülse 5 angeordneten Anschlag 20,20' mit dem an der Schubstange 4 angeordneten Steg 19,19' ist die Vorschubhülse 5 dann nicht mehr beweglich, wenn der Vorrat der Ampulle 2 vollständig aufgebraucht ist. In der Regel entspricht die mittels der Dosierhülse vorgewählte und mittels Dosisaufdruck 9 abgelesene Dosis der an der Dosisskala 21 abgelesenen möglichen zu verabreichenden Dosis vollständig. Für den Fall aber, dass bei abnehmendem Ampullenvorrat dieser Vorrat nicht mehr ausreicht die eingestellte und vorgewählte Dosis voll zu verabreichen, kann dieser Mangel mittels der Dosisskala 21 erkenntlich gemacht werden, indem die Vorschubhülse 5 nur noch einen Bruchteil des Betrages zum Laden herausgezogen werden kann, der sonst bei einer vollen Dosis möglich wäre. Der Bediener erkennt dies an einer Diskrepanz hinsichtlich der abgelesenen Dosiseinstellung der Dosierhülse mit dem abgelesenen Dosisaufdruck 9 und der Anzeige an der Dosisskala 21, die für diesen Fall immer kleiner ist.

Die Funktionsweise des erfindungsgemässen Injektionsgeräts ist folgende:

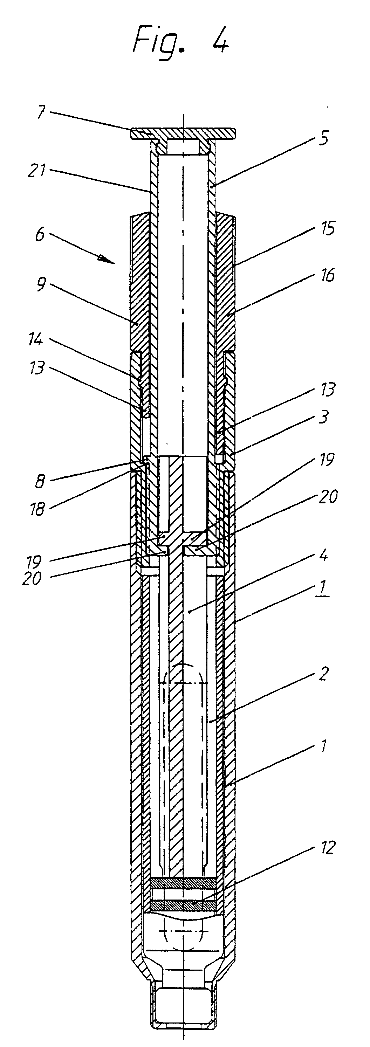

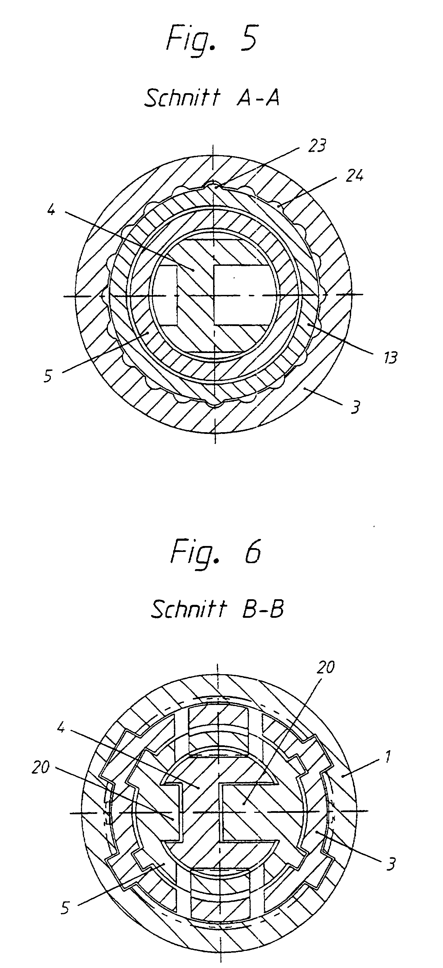

Der Bediener dreht die Dosierhülse 6 mit dem Treppenglied 13 solange, bis der gewünschte Dosisaufdruck 9 durch den Markierpfeil 22 angezeigt wird. Dann wird das Injektionsgerät geladen. Fig. 1 zeigt dieses vor dem Laden. Zum Laden wird die Vorschubhülse 5 durch den mit ihr mechanisch verbundenen Deckel 7 nach oben gezogen, bis der Nocken 8, der Bestandteil der Vorschubhülse 5 ist, gegen die aktuell eingestellte Treppenstufe des Treppenglieds 13 anschlägt. Dabei gleiten die Rastnocken 10 der Vorschubhülse 5 über die Verzahnungen 17 der Schubstange 4, deren Zähne (nicht dargestellt) nach unten gepfeilt sind. Um zu verhindern, dass die Schubstange 4 dennoch dabei nach oben geschoben wird sind die Rasten 11 am Mechanikhalter 3 vorgesehen, der nicht längsverschiebar ist. Somit stemmen sich die Rasten 11 in die Verzahnungen 17 und verhindern ein nach oben Bewegen der Schubstange 4. Zum Verabreichen der vorgewählten Dosis wird vom Bediener Druck auf den Deckel 7 und auf die mit ihm verbundene Vorschubhülse 5 in Richtung nach unten ausgeübt. Dabei stemmen sich die Rastnocken 10 in die Verzahnungen 17 und nehmen die Schubstange 4 quasi mit und der mit der Schubstange 4 verbundene Stopfen 12 übt Druck auf die im Ampullenhalter 1 befindliche Ampulle 2 aus, wodurch die flüssige Substanz appliziert wird, indem sie durch eine nicht dargestellte Injektionsnadel verabreicht wird. Beim Niederdrücken der Vorschubhülse 5 mittels des Deckels 7 gleiten die Zähne der Verzahnungen 17 auch über die Rasten 11 des Mechanikhalters 3, wobei diese durch die gewählte Pfeilung der Zähne der Verzahnungen 17 ausser Eingriff sind. Die Vorschubbewegung der Vorschubhülse 5 in Richtung nach unten dauert solange an, bis der Nocken 8 gegen die Schulter 18 des Mechanikhalters 3 anschlägt, so dass die vorgewählte Dosis der flüssigen Substanz verabreicht ist. Durch erneutes Laden ist das Injektionsgerät für einen oder mehrere nachfolgende Injektionsvorgänge bereit. Die Schubstange 4 verharrt bei den Ladevorgängen im unmittelbar zuvor gegebenen Zustand durch die Verzahnung von Rasten 11 mit den Zähnen der Verzahnungen 17. Insbesondere bewegt sich die Schubstange 4 nur in eine Richtung, nämlich nach unten, während die Vorschubhülse 5 sich in zwei Richtungen bewegen lässt, nämlich zum Laden nach oben und zum Applizieren nach unten. Diese bidirektionale Längsverschiebbarkeit der Vorschubhülse 5 stösst dann auf eine Grenze, wenn, wie in Figur 4 dargestellt, der Vorrat der Ampulle 2 durch den unmittelbar vorhergehenden Injektionsvorgang vollständig aufgebraucht ist. Dann liegen Steg 19,19' und Anschlag 20,20' vollständig aneinander. Ein Zurückziehen der Vorschubhülse 5 für einen erneuten Ladevorgang ist dann nicht mehr möglich. Auf diese Weise wird verhindert, dass bei leerer Ampulle 2 keine weitere Injizierung mit möglicherweise gravierenden gesundheitlichen Folgen entstehen kann. Für den Fall, dass, je nach Dosiswahl der Vorrat in der Ampulle 2 dieser Dosiseinstellung nur noch zu einem Bruchteil genügt, dadurch dass die Ampulle 2 fast leer ist, ist die an der Vorschubhülse 5 angebrachte Dosisskala 21 vorgesehen. Durch den zuvor beschriebenen Mechanismus mit dem Steg 19,19' und dem Zusammenwirken mit dem Anschlag 20,20' lässt sich in diesem Fall die Vorschubhülse nur noch um den Bruchteil einer vollen voreingestellten Dosis zum Laden herausziehen, bis der Anschlag 20,20' vom Steg 19,19' gestoppt wird. An der Dosisskala 21 erscheint ein geringerer zu verabreichender Betrag an Dosismenge als durch den Dosisaufdruck 9 mittels Markierpfeil 22 ablesbar ist. Bei der Dosisskala 21 dient als Ablesemarkierung der obere Rand des Bedienelements 15. Durch diese Diskrepanz wird dem Benutzer erkenntlich gemacht, dass der in der Ampulle noch vorhandene Restbetrag keiner vollen vorgewählten Dosis entspricht und er somit möglicherweise gravierende gesundheitliche Folgen vermeiden kann. Gemäss der Figuren 5 und 6 weist die Schubstange 4 im wesentlichen einen H-förmigen Querschnitt auf. Der Mechanikhalter 3 weist in seinem Innern eine Vielzahl von Ausbuchtungen 24 auf, in die Vorsprünge 23 des Treppenglieds 13 einrasten (Fig. 5). Die Länge des Injektionshubs X ist durch die einzelnen Differenzen der Treppenstufen des Treppeglieds 13 so festgelegt, dass bei einer Ampulle 2 mit einem gegebenen Durchmesser, X ein ganzzahliges Vielfaches einer Dosiseinheit ist. Ausserdem ist der Steg 19,19' so angebracht an der Schubstange 4, dass bei dessen Anliegen am Anschlag 20,20' die Ampulle 2 vollständig entleert ist.

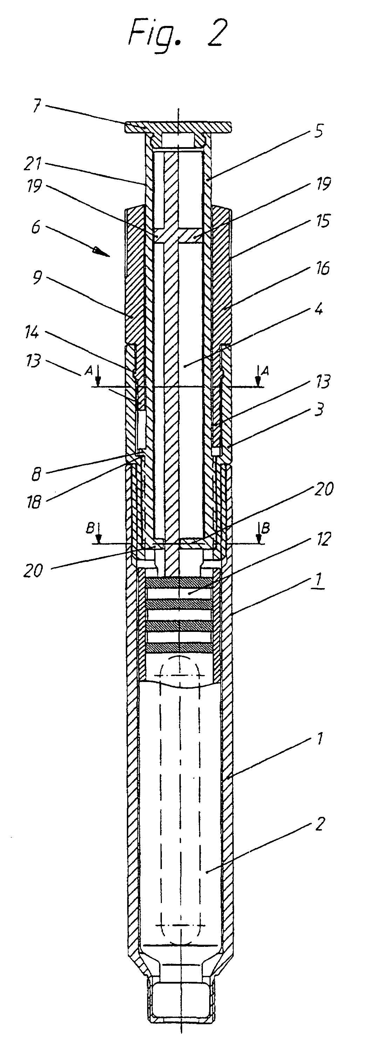

Die Fig. 2 zeigt das Injektionsgerät in einer Schnittdarstellung, die gegenüber der in Figur 2 gezeigten Darstellung um 90 Grad gedreht ist. Dabei sind alle Bezugszeichen mit denen der Fig. 1 identisch. Die Fig. 3 zeigt das Injektionsgerät in Vorderansicht. Fig. 4 zeigt einen Schnitt gemäss Fig. 2 mit der Schubstange 4 im Endanschlag durch das Anliegen von Steg 19,19' am Anschlag 20,20', so dass die Vorschubhülse 5 zum Laden nicht mehr nach hinten herausgezogen werden kann. Die Fig. 5 zeigt eine Schnittdarstellung längs der Linie A - A in Fig. 1. Dabei ist deutlich der H-förmige Querschnitt der Schubstange 4 ersichtlich. Die Fig. 6 zeigt eine Schnittdarstellung längs der Linie B - B in Fig. 1. Dabei ist zu erkennen, wie der Anschlag 20,20' mit der im Querschnitt H-förmigen Schubstange 4 zusammenwirkt.

Das erfindungsgemässe Injektionsgerät kann zum Verabreichen von Medikamenten dienen. Es können aber auch Lotionen und andere flüssige Substanzen damit verabreicht werden.

## Patentansprüche

1. Injektionsgerät zum Injizieren einer vorwählbaren Dosis einer flüssigen Substanz aus einer in einem Ampullenhalter (1) befindlichen Ampulle (2) mit einem Stopfen (12) und mit einem mit diesem mechanisch verbundenen hülsenförmigen Mechanikhalter (3), in dessen Innern eine längsverschiebbare Schubstange (4) vorgesehen ist, die auf die Ampulle (2) einwirkt, wobei die Schubstange (4) von einer mit dieser mechanisch gekoppelten, ebenfalls längsverschiebbaren Vorschubhülse (5) umgeben ist, wobei ein Dosisaufdruck (9) zum Ablesen einer zu injizierenden Dosis vorgesehen ist und ein Mechanismus (19, 20) vorgesehen ist, der ein Laden des Injektionsgeräts durch Herausziehen der Schubstange (4) dann verhindert, wenn der Vorrat der Ampulle (2) vollständig aufgebraucht ist,
**dadurch gekennzeichnet, dass**
zum Vorwählen der zu injizierenden Dosis eine Dosierhülse (6) vorgesehen ist; und dass der Dosisaufdruck (9) an einem zylindrischen Teil der Dosierhülse (6) vorgesehen ist, der mit einem Markierpfeil (22) am Mechanikhalter (3) zusammenwirkt, so dass die eingestellte Dosis ablesbar ist, wobei die tatsächlich mögliche verabreichbare Dosis an einer im Bereich des oberen Endes der Vorschubhülse (5) angebrachten Dosisskala (21) ablesbar ist und der Mechanismus einen im unteren Endbereich der Vorschubhülse (5) angeordneten Anschlag (20, 20') und einen damit zusammenwirkenden, an der Schubstange (4) angeordneten Steg (19, 19') umfasst.

2. Injektionsgerät nach Anspruch 1, **dadurch gekennzeichnet, dass** die Dosierhülse (6) mit einem Treppenglied (13) versehen ist, das vorzugsweise nach hinten orientiert ist.

3. Injektionsgerät nach Anspruch 2, **dadurch gekennzeichnet, dass** die Dosierhülse (6) am Mechanikhalter (3) in dessen oberen Endbereich angeordnet ist, wobei durch eine Drehbewegung der Dosierhülse (6) mit dem Treppenglied (13) die jeweils zu verabreichende Dosis einstellbar ist.

4. Injektionsgerät nach Anspruch 2 oder 3, **dadurch gekennzeichnet, dass** die Dosierhülse (6) mit dem Treppenglied (13) die Vorschubhülse (5) umgibt.

5. Injektionsgerät nach einem der Ansprüche 2 - 4, **dadurch gekennzeichnet, dass** ein an der Vorschubhülse (5) angeordneter Nocken (8) beim Herausziehen der Vorschubhülse (5) in Richtung des Deckels (7) gegen die jeweils eingestellte Treppenstufe des Treppenglieds (13) anschlägt und beim Niederdrücken der Vorschubhülse (5) der Nocken (8) gegen eine Schulter (18) des Mechanikhalters (3) anschlägt, wodurch sich der Injektionshub X ergibt.

6. Injektionsgerät nach einem der Ansprüche 1 bis 5, **dadurch gekennzeichnet, dass** zur mechanischen Kopplung von Schubstange (4) mit Vorschubhülse (5) die Schubstange (4) mit zwei gegenüberliegenden Verzahnungen (17) versehen ist, die mit zwei Rastnocken (10) der Vorschubhülse (5) zusammenwirken.

7. Injektionsgerät nach Anspruch 6, **dadurch gekennzeichnet, dass** der Mechanikhalter (3) im Bereich seines unteren Endes zwei Rasten (11) aufweist, die mit den Verzahnungen (17) der Schubstange (4) zusammenwirken.

8. Injektionsgerät nach Anspruch 6 oder 7, **dadurch gekennzeichnet, dass** die Zahnhöhe der Zähne der Verzahnungen (17) an der Schubstange (4) einer Stufenhöhe des Treppenglieds (13) entsprechen.

9. Injektionsgerät nach einem der Ansprüche 2 bis 8, **dadurch gekennzeichnet, dass** die Dosierhülse (6) zusammengesetzt ist aus einem Bedienelement (15) mit einer Profilierung, einem daran anschliessenden zylindrischen Teil (16) mit dem Dosisaufdruck (9), einem Einrastanschlag (14) und dem daran sich anschliessenden Treppenglied (13).

10. Injektionsgerät nach einem der Ansprüche 2 bis 9, **dadurch gekennzeichnet, dass** der Mechanikhalter (3) in seinem Innern eine Vielzahl von Ausbuchtungen (24) aufweist, in die Vorsprünge (23) des Treppenglieds (13) einrasten.

11. Injektionsgerät nach einem der Ansprüche 1 bis 10, **dadurch gekennzeichnet, dass** die Schubstange (4) im wesentlichen einen H-förmigen Querschnitt hat.

12. Injektionsgerät nach einem der Ansprüche 1 bis 11, gekennzeichnet zur Verabreichung von Medikamenten.

13. Injektionsgerät nach einem der Ansprüche 1 bis 12, **dadurch gekennzeichnet, dass** der Mechanikhalter (3) an seinem äusseren Umfang eine Dosisskala (21) aufweist.

## Claims

1. Injection device for injecting a preselectable dose of a liquid substance from an ampoule (2) contained in an ampoule holder (1), having a stopper (12) and a sleeve-shaped mechanical system holder (3) linked mechanically thereto, inside which there is provided a longitudinally displaceable push-rod (4) which operates on the ampoule (2), the push-rod (4) being surrounded by a likewise longitudinally displaceable feeder sleeve (5) which is mechanically coupled thereto, a dosage mark (9) being provided in order to read off a dose to be injected, and a mechanism (19, 20) being provided which prevents the injection device from being loaded by withdrawing the push-rod (4) once the supply in the ampoule (2) has all been expended,
**characterised in that**
a dosing sleeve (6) is provided for preselection of the dose to be injected; and that the dosage mark (9) is provided on a cylindrical portion of the dosing sleeve (6) which cooperates with a marker arrow (22) on the mechanical system holder (3) so as to enable the set dose to be read off, it being possible to read off the dosage that actually can be administered from a dosage scale (21) positioned near the top end of the feeder sleeve (5), and the mechanism comprising a limit stop (20, 20') disposed in the lower end portion of the feeder sleeve (5) and a cross-piece (19, 19') which cooperates therewith and is disposed on the push-rod (4).

2. Injection device according to claim 1, **characterised in that** the dosing sleeve (6) is provided with a stepped element (13) which is preferably orientated towards the rear.

3. Injection device according to claim 2, **characterised in that** the dosing sleeve (6) is arranged on the mechanical system holder (3), in the top end portion thereof, thereby enabling the respective dose that is to be administered to be set by means of a rotational movement of the dosing sleeve (6) with the stepped element (13).

4. Injection device according to claim 2 or 3, **characterised in that** the dosing sleeve (6) with the stepped element (13) surrounds the feeder sleeve (5).

5. Injection device according to any of claims 2 to 4, **characterised in that** when the feeder sleeve (5) is withdrawn in the direction of the cover (7), a cam (8) arranged on the feeder sleeve (5) strikes the particular graduation that has been set on the stepped element (13), and when the feeder sleeve (5) is depressed, the cam (8) strikes a shoulder (18) of the mechanical system holder (3), thereby resulting in the injection stroke X.

6. Injection device according to any of claims 1 to 5, **characterised in that** for mechanically coupling the push-rod (4) with the feeder sleeve (5), the push-rod (4) is provided with two opposing sets of teeth (17) which cooperate with two locking cams (10) belonging to the feeder sleeve (5).

7. Injection device according to claim 6, **characterised in that** near its lower end the mechanical system holder (3) incorporates two notches (11) which cooperate with the sets of teeth (17) on the push-rod (4).

8. Injection device according to claim 6 or 7, **characterised in that** the height of the teeth of the sets of teeth (17) on the push-rod (4) match the height of a step on the stepped element (13).

9. Injection device according to any of claims 2 to 8, **characterised in that** the dosing sleeve (6) is made up of a profiled control element (15), an adjoining cylindrical part (16) incorporating the dosage mark (9), a latch-type stop (14) and the stepped element (13) adjoining the latter.

10. Injection device according to any of claims 2 to 9, **characterised in that** in its interior the mechanical system holder (3) has a large number of bays (24) into which projections (23) on the stepped element (13) engage.

11. Injection device according to any of claims 1 to 10, **characterised in that** the push-rod (4) is substantially H-shaped in cross-section.

12. Injection device according to any of claims 1 to 11, characterised for administering medicines.

13. Injection device according to any of claims 1 to 12, **characterised in that** the mechanical system holder (3) incorporates a dosage scale (21) on its external circumference.

## Revendications

1. Dispositif d'injection pour injecter une dose présélectionnable d'une substance fluide à partir d'une ampoule (2) logée dans un porte-ampoule (1) avec un tampon (12) et avec un support mécanique (3) en forme de manchon relié mécaniquement avec ledit porte-ampoule, à l'intérieur duquel est prévue une tige de poussée (4) coulissante longitudinalement qui coopère avec l'ampoule (2), où le piston (4) est entouré par un manchon d'entraînement (5) également coulissant longitudinalement et accouplé mécaniquement avec celui-ci, où est prévue une empreinte de dosage (9) pour la lecture de la dose à injecter ainsi qu'un mécanisme (19, 20) qui empêche ensuite un chargement du dispositif d'injection par extraction du piston (4) quand la réserve de l'ampoule (2) est complètement épuisée, **caractérisé en ce qu'**il comporte une bague de dosage (6) pour la présélection de la dose à injecter et **en ce que** l'empreinte de dosage est prévue sur une partie cylindrique de la bague de dosage (6) et coopère avec une flèche de référence (22) sur le support mécanique (3), de manière que la dose sélectionnée soit lisible, tandis que la dose effective qui peut être effectivement administrée est lisible sur une échelle de dosage (21) appliquée en correspondance de l'extrémité supérieure du manchon d'entraînement (5) et le mécanisme comprenant une butée (20, 20') disposée dans la partie d'extrémité inférieure du manchon d'entraînement (5) et une saillie (19, 19') disposée sur la tige de poussée (4), coopérant avec ladite butée.

2. Dispositif d'injection selon la revendication 1, **caractérisé en ce que** la bague de dosage (6) est pourvue d'un élément à gradins (13), qui est de préférence orienté vers l'arrière.

3. Dispositif d'injection selon la revendication 2, **caractérisé en ce que** la bague de dosage (6) sur le support mécanique (3) est disposée dans la partie d'extrémité supérieure de celui-ci, tandis que la dose qui peut être administrée est réglable par un mouvement de rotation de la bague de dosage (6) avec l'élément à gradins (13).

4. Dispositif d'injection selon la revendication 2 ou 3, **caractérisé en ce que** la bague de dosage (6) avec l'élément à gradins (13) entoure le manchon d'entraînement (5).

5. Dispositif d'injection selon une des revendications 2 - 4, **caractérisé en ce qu'**une saillie (8) disposée sur le manchon d'entraînement (5) vient en butée contre le gradin actuellement sélectionné durant l'extraction du manchon d'entraînement (5) en direction du bouchon (7) et **en ce que** la saillie (8) vient en butée contre un épaulement (18) du support mécanique (3) durant l'introduction du manchon d'entraînement (5), en déterminant ainsi la course d'injection X.

6. Dispositif d'injection selon une des revendications 1 à 5, **caractérisé en ce que**, pour l'accouplement mécanique de la tige de poussée (4) avec le manchon d'entraînement (5), la tige de poussée (4) est pourvue de deux dentures opposées (17) qui coopèrent avec deux ergots d'arrêt (10) du manchon d'entraînement (5).

7. Dispositif d'injection selon la revendication 6, **caractérisé en ce que** le support mécanique (3) comporte deux ergots (11) en correspondance de son extrémité inférieure, lesquels coopèrent avec les dentures (17) de la tige de poussée (4).

8. Dispositif d'injection selon la revendication 6 ou 7, **caractérisé en ce que** les hauteurs des dents des dentures (17) sur la tige de poussée (4) correspondent aux hauteurs des gradins de l'élément à gradins (13).

9. Dispositif d'injection selon une des revendications 2 à 8, **caractérisé en ce que** la bague de dosage (6) se compose d'un élément de manoeuvre (15) avec un profilage, une partie cylindrique (16) adjacente avec l'empreinte de dosage (9), un taquet de verrouillage (14) et l'élément à gradins (13) adjacent.

10. Dispositif d'injection selon une des revendications 2 à 9, **caractérisé en ce que** le support mécanique (3) présente à l'intérieur une pluralité de cavités (24) dans lesquelles s'enclenchent des saillies (23) de l'élément à gradins (13).

11. Dispositif d'injection selon une des revendications 1 à 10, **caractérisé en ce que** la tige de poussée (4) a essentiellement une section en forme de H.

12. Dispositif d'injection selon une des revendications 1 à 11, **caractérisé en ce qu'**il est destiné à l'administration de médicaments.

13. Dispositif d'injection selon une des revendications 1 à 12, **caractérisé en ce que** le support mécanique (3) comporte une échelle de dosage (21) sur sa circonférence extérieure.
